# EUROPEAN PATENT SPECIFICATION

(11) **EP 3 499 229 B1**
(45) Date of publication and mention of the grant of the patent: **01.12.2021**
(21) Application number: 18211529.5
(22) Date of filing: 11.12.2018
(51) Int. Cl.: G01N 33/49, G01N 35/00, G01N 35/10, G01N 15/00, G01N 15/06, G01N 15/12

(54) **METHOD AND APPARATUS FOR ANALYZING BLOOD**
VERFAHREN UND VORRICHTUNG ZUR ANALYSE VON BLUT
PROCÉDÉ ET APPAREIL D'ANALYSE DE SANG

(30) Priority: 12.12.2017 JP 2017237762
(43) Date of publication of application: 19.06.2019
(73) Proprietor: Nihon Kohden Corporation, Tokyo 161-8560 (JP)
(72) Inventor: MASUDA, Koji, Tokorozawa-shi, Saitama 359-0037 (JP); HAMADA, Tatsuya, Tokorozawa-shi, Saitama 359-0037 (JP); NAKAYAMA, Ryoko, Tokorozawa-shi, Saitama 359-0037 (JP)
(74) Representative: Grünecker Patent- und Rechtsanwälte PartG mbB

(56) References cited:
- WO-A1-2017/002329
- US-A1- 2015 177 222
- US-B1- 6 784 981

## Description

### BACKGROUND

The present invention relates to a method and apparatus for analyzing blood.

In order to obtain blood information such as the counting of red blood cells, blood platelets, and white blood cells, or the classification of white blood cells, a blood analyzing apparatus is used. A blood analyzing apparatus has a suction tube which is used for sucking blood filled in a blood collecting tube, and a plurality of measuring units which obtain information of the blood sucked by the suction tube.

In such a blood analyzing apparatus, a short sample sometimes occurs when the blood is sucked by the suction tube.

Since blood has high viscosity, there is a possibility that a short sample occurs in the tip end side of the suction tube. In this case, a required amount of blood may not be sucked, and instead air may be sucked.

When the suction tube is blocked by foreign matters, for example, a sufficient amount of blood may not be sucked, but a short sample may occur in the base end side of the suction tube. At this time, the base end side of the suction tube is not filled with the blood.

When a failure in suction of blood occurs as described above, for example, a sample in which a required amount of blood is not sucked, i.e., a blood short sample occurs may be caused in regions of the tip and base end sides of the suction tube.

When blood is analyzed by using such a sample, correct blood information cannot be acquired because a required amount of blood does not exist. Therefore, it is necessary to detect a blood short sample.

In connection with the above, a technique of detecting a blood short sample is disclosed in Patent Literature 1 below. In the invention disclosed in Patent Literature 1, blood is sucked by a suction tube, and then blood existing in the regions which are in an internal space of the suction tube, and which are on the tip and base end sides with respect to a blood analysis region used for analyzing the blood is supplied to a detecting part. The detecting part measures the opacity of the supplied blood, and detects a blood short sample based on the value of the opacity.

### [Citation List]

### [Patent Literature]

Patent Literature 1: Japanese Patent No. 4,593,404

In the above-described detecting method, however, blood to be subjected to analysis, and that to be subjected to detection of a blood short sample are supplied respectively from different regions in the internal space of the suction tube. When a blood short sample occurs in the blood analysis region, therefore, the blood short sample cannot be detected. In the case where, when a blood short sample occurs in the blood analysis region, the blood is analyzed, the blood analysis may lead to an erroneous measurement result.
US 2015/177222 A1 discloses a self-diagnosing method for measuring results from blood analyzers. US 6,784,981 B1 discloses A flow cytometry-based hematology system. WO 2017/002329 A1 discloses a method and apparatus for analyzing blood.

The present invention has been conducted in order to solve the problems. It is an object of the present invention to provide a method and apparatus for analyzing blood in which an erroneous measurement result can be prevented from being acquired.

### SUMMARY OF THE INVENTION

The invention provides a blood analyzing method and system according to the independent claims. Preferred embodiments are specified in the dependent claims.

The blood analyzing method of the present invention is a blood analyzing method in which, when blood is sucked by a suction tube, a blood short sample in the suction tube is detected, wherein the method has a determining step of, in a case where the mean corpuscular hemoglobin concentration of the blood in a blood analysis region that is in the suction tube, and that is to be subjected to analysis of the blood is lower than a predetermined threshold, determining that a blood short sample occurs.

The blood analyzing apparatus of the present invention is a blood analyzing apparatus in which, when blood is sucked by a suction tube, a blood short sample in the suction tube is detected, wherein the apparatus has a determining unit which, in a case where a mean corpuscular hemoglobin concentration of the blood in a blood analysis region that is in the suction tube, and that is to be subjected to analysis of the blood is lower than a predetermined threshold, determines that a blood short sample occurs.

According to the blood analyzing method and blood analyzing apparatus which have been described above, a blood short sample in the suction tube is detected by using blood existing in the blood analysis region. Even when a blood short sample occurs in the blood analysis region, therefore, the blood short sample can be detected. Consequently, a sample in which a blood short sample occurs in the blood analysis region can be detected, and an erroneous measurement result can be prevented from being provided.

### BRIEF DESCRIPTION OF DRAWINGS

Fig. 1 is a diagram illustrating a blood analyzing apparatus of an embodiment.
Fig. 2 is a schematic sectional view illustrating a suction tube and a washing unit.
Fig. 3 is a diagram illustrating a second measuring unit.
Fig. 4A is a schematic view illustrating a displaying unit in the case where blood is normally sucked.
Fig. 4B is a schematic view illustrating the displaying unit in the case where a blood short sample occurs.
Fig. 5 is a flowchart illustrating a blood analyzing method of the embodiment.
Fig. 6 is a chart illustrating a step of forming an air layer.
Figs 7A to 7E are charts illustrating steps of sucking blood.
Fig. 8 is a view illustrating a manner in which the user presses a command button of "REMEASUREMENT."
Fig. 9 is a view illustrating a manner in which a given display region for a specimen ID is colored.

### DETAILED DESCRIPTION OF EMBODIMENTS

An embodiment of the present invention will be described with reference to the drawings. In the description of the drawings, identical components are denoted by the same reference numerals, and duplicated description is omitted. In the drawings, the dimension ratios may be sometimes different from the actual ratios for the sake of convenience in description.

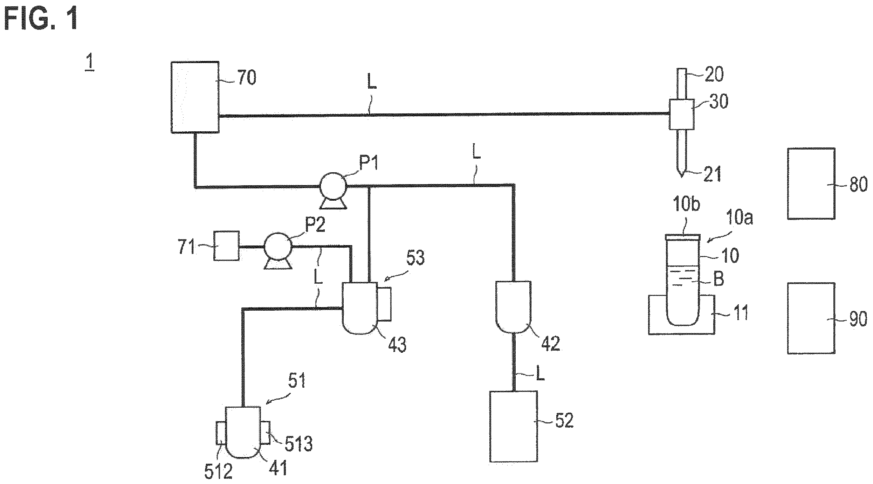

Fig. 1 is a diagram illustrating a blood analyzing apparatus 1 of the embodiment.

The blood analyzing apparatus 1 may include a blood collecting tube 10 which is to be is filled with collected blood B, a suction tube 20 which sucks the blood in the blood collecting tube 10, and a washing unit 30 which washes the suction tube 20. The blood analyzing apparatus 1 may further include three reservoirs 41, 42, 43 to which the blood is discharged out from the suction tube 20. According to the invention, the blood analyzing apparatus 1 includes a first measuring unit 51 which measures hemoglobin (HGB), a second measuring unit 52 which measures the red blood cell count (RBC) and the hematocrit value (HCT), and a third measuring unit 53 which measures the white blood cell count (WBC). The blood analyzing apparatus 1 may further include a storage unit 70 which stores a dilute solution, and a storage unit 71 which stores a hemolytic agent. The blood analyzing apparatus 1 may further include two pumps P1, P2 for delivering the dilute solution and the hemolytic agent, pipes L through which the dilute solution and the hemolytic agent pass, and electromagnetic valves (not illustrated) for opening and closing the pipes L. The blood analyzing apparatus 1 may further include a controlling unit 80 which controls the components, and a displaying unit 90 which displays various kinds of information.

In the blood collecting tube 10, an opening 10a is formed in an upper portion, and a rubber plug 10b closes the opening 10a to hermetically seal the interior of the blood collecting tube 10. The material constituting the blood collecting tube 10 is not particularly limited, and a conventionally used material may be used as the material. For example, glass, plastics, or the like may be used in the blood collecting tube 10.

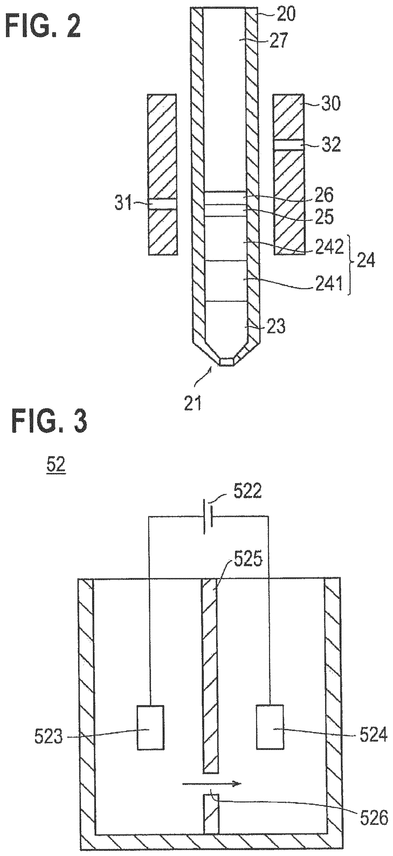

Fig. 2 is a schematic sectional view illustrating the suction tube 20 and the washing unit 30.

The suction tube 20 has a needle-shaped puncturing portion 21 on the tip end side. The puncturing portion 21 punctures the rubber plug 10b of the blood collecting tube 10, the suction tube 20 is inserted into the blood collecting tube 10, and, in this state, the suction tube 20 sucks the blood in the blood collecting tube 10.

A flow path in which the sucked blood is stored is formed in the suction tube 20. The flow path may include a first waste region 23 where waste blood is stored, a blood analysis region 24 which is used for analyzing blood, and a second waste region 25 where waste blood is stored, in this order starting from the lower portion of Fig. 2. The flow path may further include an air layer region 26 in which an air layer is formed, and a dilute solution region 27 in which the dilute solution is placed. In the drawings, the respective regions in the flow path are clearly separated and distinguished from each other. However, there is no particular structure for physically separating these regions from each other. Particularly, the first waste region 23, the blood analysis region 24, and the second waste region 25 simply represent the order of blood which is placed in the flow path when the blood is first sucked, and which is then used or wasted by an adequate amount in each of post steps. Therefore, the first waste region 23, the blood analysis region 24, the second waste region 25, the air layer region 26, and the dilute solution area 27 are properly shifted in a flow direction (the vertical direction in Fig. 2) of the flow path, in respective steps of the blood analyzing method that will be described later.

The blood analysis region 24 is further divided in the following manner. The blood analysis area 24 may include: a first region 241 in which blood to be used for measuring hemoglobin and the white blood cell count is stored; and a second region 242 in which blood to be used for measuring the red blood cell count and the hematocrit value is stored. The blood analysis region 24 may further include a region in which blood to be used in three-category classification of white blood cells or five-category classification of white blood cells is stored. Here, the three-category classification of white blood cells means a process in which white blood cells is fractionated into granulocytes, lymphocytes, and monocytes, and the fractionated cells are counted, and the five-category classification of white blood cells means a process in which white blood cells is fractionated into neutrophils, eosinophils, and basophils, and lymphocytes and monocytes, and the fractionated cells are counted.

The material constituting the suction tube 20 is not particularly limited, and, for example, the suction tube is made of a metal such as a stainless steel alloy.

As illustrated in Fig. 2, the washing unit 30 is placed so as to be slidable relative to the outer circumference of the suction tube 20. The washing unit 30 may include two passing portions 31, 32 through which the dilute solution can pass. The passing portions 31, 32 are placed so as to be laterally paired, and located at different heights.

Returning to Fig. 1, the blood analyzing apparatus 1 may further include the first reservoir 41, the second reservoir 42, and the third reservoir 43.

The blood stored in the first region 241 of the suction tube 20 is ejected into the first reservoir 41 and the third reservoir 43. The blood stored in the first region 241 of the suction tube 20 is ejected into the first reservoir 41 through the third reservoir 43. The blood stored in the second region 242 of the suction tube 20 is ejected into the second reservoir 42.

For example, quartz, glass, or a synthetic resin can be used as the material constituting the reservoirs 41, 42, 43.

The blood analyzing apparatus 1 may further include the first measuring unit 51, the second measuring unit 52, and the third measuring unit 53.

The first measuring unit 51 is coupled to the third reservoir 43 through the pipe L. In the first measuring unit 51, hemoglobin in the first reservoir 41 is measured by using the blood ejected into the first reservoir 41.

As illustrated in Fig. 1, the first measuring unit 51 may include a light emitting element 512 and a light detecting element 513. The light emitting element 512 and the light detecting element 513 are placed so as to be opposed to the first reservoir 41.

For example, the light emitting element 512 is an LED, and the light detecting element 513 is a photodiode. However, the kinds of the elements are not particularly limited.

The light emitting element 512 emits light to the sample in the first reservoir 41. The light detecting element 513 detects the transmitted light which is transmitted through the sample, to measure the intensity of the transmitted light. Hemoglobin of the sample in the first reservoir 41 is measured based on the intensity of the light emitted from the light emitting element 512, and that of the transmitted light.

The second measuring unit 52 is coupled to the second reservoir 42 through the pipe L. In the second measuring unit 52, the red blood cell count and the hematocrit value are measured by using the blood ejected into the second reservoir 42. The electrical resistance method is used in the second measuring unit 52.

Fig. 3 is a diagram illustrating the second measuring unit 52.

As illustrated in Fig. 3, the second measuring unit 52 may include a resistance detecting section 522, a pair of electrodes 523, 524, and a chamber 525. In the second measuring unit 52, when blood cells are passed through an aperture 526 which is disposed between the two electrodes 523, 524, the electrical resistance between the two electrodes 523, 524 is changed in accordance with the sizes of the blood cells. The red blood cell count and the hematocrit value are measured based on the number and widths of pulses of the resistance change.

The third measuring unit 53 measures the white blood cell count by using the blood ejected into the third reservoir 43. In the same or similar manner as the second measuring unit 52, the third measuring unit 53 measures the white blood cell count by the electrical resistance method.

The storage unit 70 is a tank which stores the dilute solution. The dilute solution which is stored in the storage unit 70 is used for washing of the suction tube 20, dilution of the bloods which are ejected into the second reservoir 42 and the third reservoir 43, and the like. The dilute solution is supplied to the second reservoir 42 and the third reservoir 43 by the pump P1.

The storage unit 71 is a tank which stores the hemolytic agent for measuring the white blood cell count. The hemolytic agent which is stored in the storage unit 71 is supplied to the third reservoir 43 to dissolve and stabilize red blood cells of the blood in the third reservoir 43. This enables the sample to be subjected to the measurements of the white blood cell count and hemoglobin. The hemolytic agent in the storage unit 71 is supplied into the third reservoir 43 by the pump P2.

The controlling unit 80 is, for example, a CPU, and executes controls of the above-described components, various calculations, and the like in accordance with programs. For example, the controlling unit 80 functions as a calculating unit and a determining unit.

The calculating unit calculates the mean corpuscular hemoglobin concentration (MCHC) based on the hemoglobin measured by the first measuring unit 51, and the hematocrit value measured by the second measuring unit 52. The mean corpuscular hemoglobin concentration indicates the concentration of hemoglobin contained in one red blood cell, and is calculated by hemoglobin (g/dl) ÷ hematocrit value (%) × 100.

In the case where the mean corpuscular hemoglobin concentration is lower than a predetermined threshold, the determining unit determines that a blood short sample occurs. The method of determining a blood short sample will be described in detail later.

The supplies of the dilute solution and the hemolytic agent are performed by controls in which the controlling unit 80 causes the pumps PI, P2 to operate. When the dilute solution or the hemolytic agent flows through the predetermined pipe L, the controlling unit 80 controls the electromagnetic valve placed in the predetermined pipe L to open.

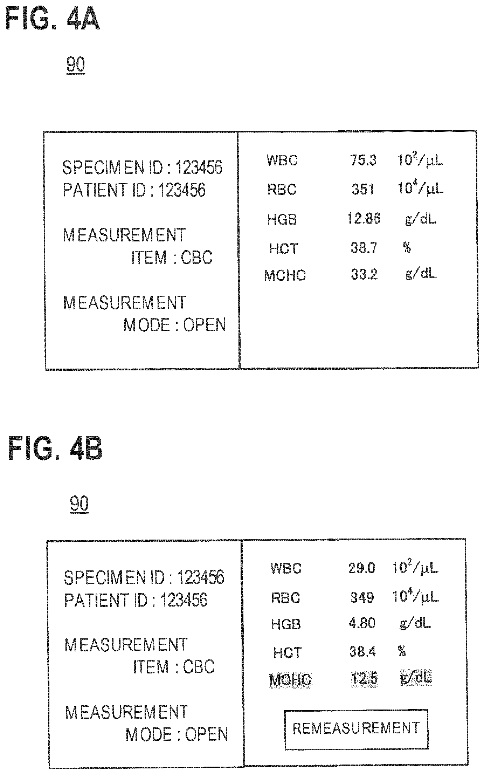

Fig. 4A is a schematic view illustrating an example of the displaying unit 90 in the case where blood is normally sucked, and Fig. 4B is a schematic view illustrating an example of the displaying unit 90 in the case where a blood short sample occurs.

The displaying unit 90 displays various kinds of information. As illustrated in Figs. 4A and 4B, for example, the white blood cell count (WBC), the red blood cell count (RBC), hemoglobin (HGB), the hematocrit value (HCT), the mean corpuscular hemoglobin concentration (MCHC), and the like are displayed on the displaying unit 90. In the case where a blood short sample occurs and the mean corpuscular hemoglobin concentration is lower than the predetermined threshold, as illustrated in Fig. 4B, the numerals of the mean corpuscular hemoglobin concentration are clearly indicated by highlight on the displaying unit 90, and a command button of "REMEASUREMENT" is displayed on a lower portion of the displaying unit 90.

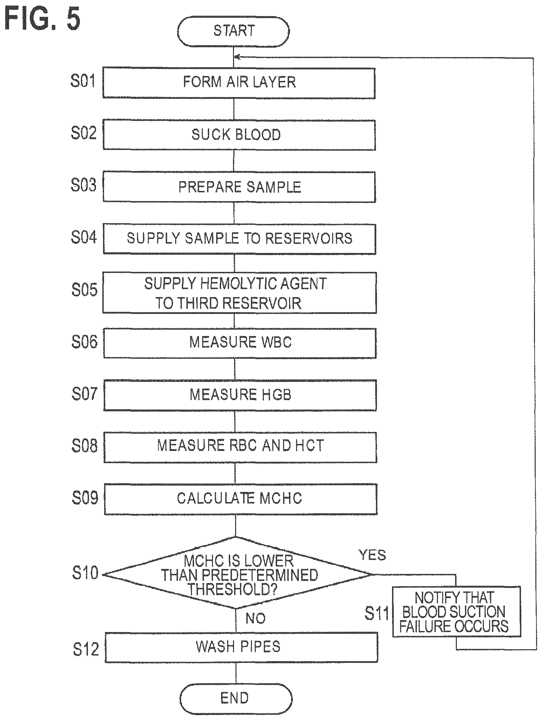

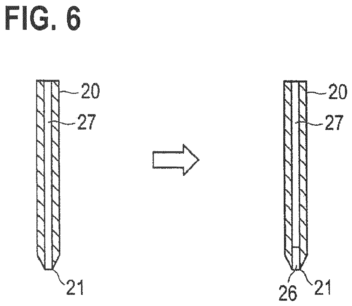

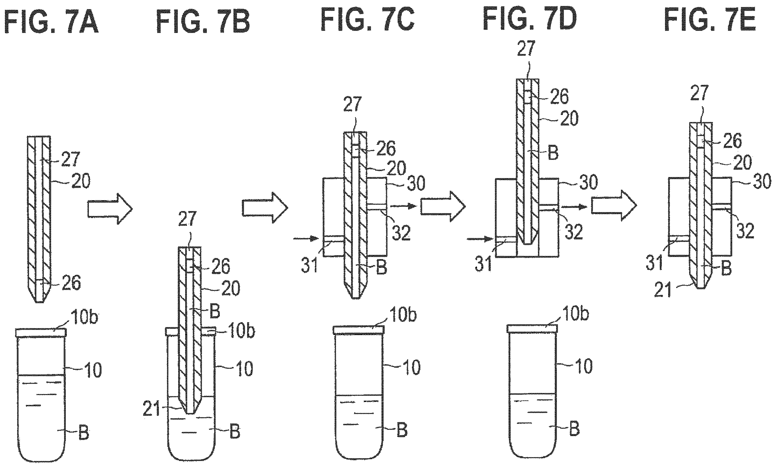

Referring to Figs. 5 to 7, next, a blood analyzing method of the embodiment will be described. Fig. 5 is a flowchart illustrating the blood analyzing method of the embodiment, Fig. 6 is a chart illustrating a step of forming the air layer, and Fig. 7 is a chart illustrating steps of sucking blood. The step illustrated in Fig. 5 can be realized by controls in which the controlling unit 80 causes the components of the blood analyzing apparatus 1 to operate. Hereinafter, the procedure of Fig. 5 will be described by appropriately referring also to Figs. 6 and 7.

First, the air layer (corresponding to the air layer region 26 in Fig. 2) is formed in the tip end side of the suction tube 20 (S01). In the suction tube 20 which is initially filled only with the dilute solution, the air layer region 26 is formed by pulling up the dilute solution region 27 as illustrated in Fig. 6. At this time, for example, the air layer region 26 of 5 µL is formed.

Next, the blood in the blood collecting tube 10 is sucked by the suction tube 20 (S02). With reference to Fig. 7, substeps of step S02 will be described in detail.

As illustrated in (A) of Fig. 7, first, the suction tube 20 in which the air layer region 26 is formed in step S01 is set above the blood collecting tube 10 that is filled with the blood B.

As illustrated in (B) of Fig. 7, next, the puncturing portion 21 of the suction tube 20 punctures the rubber plug 10b which seals the blood collecting tube 10, and the blood B is sucked into the flow path in the suction tube 20. As a result, the first waste region 23, the blood analysis region 24, and the second waste region 25 are filled with the blood B (see Fig. 2). At this time, for example, the blood B of 15.75 µL is sucked into the suction tube 20.

As illustrated in (C) of Fig. 7, next, the suction tube 20 is drawn out from the blood collecting tube 10. At this time, the dilute solution is supplied from the storage unit 70 to the passing portion 31 of the washing unit 30, and then discharged from the passing portion 32. Therefore, the dilute solution is supplied to the outer circumference of the suction tube 20, the outer circumference of the suction tube 20 is washed, and blood which is adhered thereto during the sucking step is removed.

As illustrated in (D) of Fig. 7, next, the dilute solution is supplied to the passing portion 31, and then discharged from the passing portion 32. In the blood in the suction tube 20, simultaneously, the blood placed in the first waste region 23 is discarded (see Fig. 2). At this time, for example, blood of 1 µL is is discarded.

As illustrated in (E) of Fig. 7, next, the puncturing portion 21 of the suction tube 20 is projected from the washing unit 30 in the downward direction. In the above, the substeps of step S02 have been described.

Then, samples which are to be subjected to the blood analysis are prepared (S03). In step S03, a sample which is to be subjected to the measurements of hemoglobin and the white blood cell count, and that which is to be subjected to the measurements of the red blood cell count and the hematocrit value are prepared.

Next, the samples which are prepared in step S03 are supplied to the second reservoir 42 and the third reservoir 43 (S04). For example, the sample of only 5 µL is supplied to the second reservoir 42, and the sample of only 75 µL is supplied to the third reservoir 43. The sample which is supplied to the third reservoir 43 is further supplied to the first reservoir 41.

Next, the hemolytic agent which is stored in the storage unit 71 is supplied to the third reservoir 43 (S05). The hemolytic agent which is supplied to the third reservoir 43 dissolves and stabilizes red blood cells of the blood in the third reservoir 43. This enables the sample to be subjected to the measurements of the white blood cell count and hemoglobin.

Next, the white blood cell count of the sample in the third reservoir 43 is measured by the third measuring unit 53 (S06). In the third measuring unit 53, the white blood cell count is measured by using the above-described electrical resistance method. In this step, namely, the white blood cell count is measured by using the blood which is stored in the first region 241 of the blood analysis region 24. Then, the measurement data of the white blood cell count are transmitted to the controlling unit 80, and displayed on the displaying unit 90 (see Fig. 4A).

Next, hemoglobin of the sample in the first reservoir 41 is measured by the first measuring unit 51 (the first measuring step: S07). In this step, namely, hemoglobin is measured by using the blood which is stored in the first region 241 of the blood analysis region 24. The light detecting element 513 transmits the intensity data of the detected light, to the controlling unit 80. Then, the controlling unit 80 calculates hemoglobin of the sample in the first reservoir 41, based on the intensity data of the light detected by the light detecting element 513. The hemoglobin calculated by the controlling unit 80 is displayed on the displaying unit 90 (see Fig. 4A).

Next, the sample in the second reservoir 42 is supplied to the second measuring unit 52, and the red blood cell count and the hematocrit value are measured in the second measuring unit 52 by using the above-described electrical resistance method (the second measuring step: S08). In this step, namely, the red blood cell count and the hematocrit value are measured by using the blood which is stored in the second region 242 of the blood analysis region 24. The red blood cell count and the hematocrit value are transmitted to the controlling unit 80, and displayed on the displaying unit 90 (see Fig. 4A).

Next, the mean corpuscular hemoglobin concentration is calculated by using the hemoglobin measured in step S07 and the hematocrit value measured in step S08 (the calculating step: S09). At this time, the controlling unit 80 functions as the calculating unit. Then, the value of the mean corpuscular hemoglobin concentration calculated by the calculating unit is displayed on the displaying unit 90 (see Fig. 4A).

Next, it is determined whether a blood short sample occurs or not (the determining step: S10). At this time, the controlling unit 80 functions as the determining unit. In this step, it is determined whether the mean corpuscular hemoglobin concentration calculated in step S09 is lower than the predetermined threshold or not.

As the range of the threshold, the upper limit may be adequately set from the view point that a normal state in which a blood short sample does not occur is prevented from being detected, and the lower limit may be adequately set from the view point that an occurrence of a blood short sample is prevented from being overlooked. As such a threshold, for example, the mean corpuscular hemoglobin concentration may be in a concentration range of 25% to 30%. The threshold is not limited the above-described values.

If the mean corpuscular hemoglobin concentration is higher than the predetermined threshold (S10: NO), it is determined that blood is normally sucked, and the dilute solution is supplied from the storage unit 70 to the pipe L to wash the pipe L (S12).

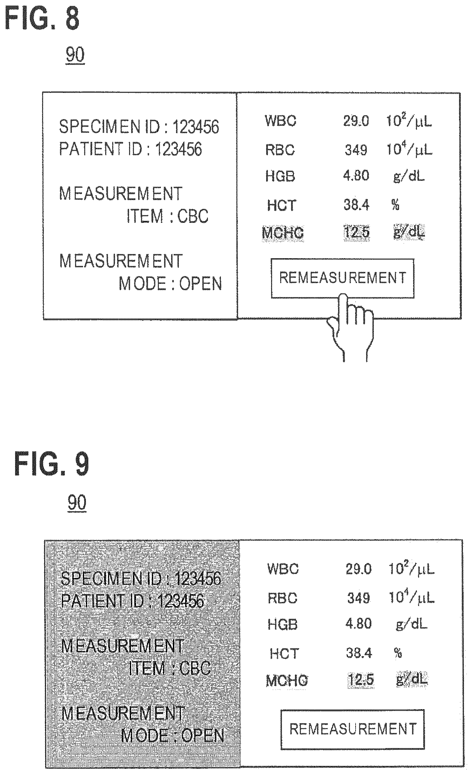

By contrast, if the mean corpuscular hemoglobin concentration is lower than the predetermined threshold (S10: YES), it is determined that a blood short sample occurs. When, after a process of step S11 is performed, the user presses the command button of "REMEASUREMENT" displayed on the displaying unit 90 as illustrated in Fig. 8, the process returns to step S01, and the mean corpuscular hemoglobin concentration is again measured.

Alternatively, the blood analyzing apparatus may be previously set so that, when the mean corpuscular hemoglobin concentration is lower than the predetermined threshold (S10: YES), and it is determined that a blood short sample occurs, the process of the controlling unit 80 may cause the remeasurement to, after the process of step S11, be automatically performed even when the user does not press the command button of "REMEASUREMENT."

The user may previously set the blood analyzing apparatus so that, in the case where the process is to return to step S01 in the remeasurement process, the user is requested to manually again set the ID of the specimen and the measurement conditions, or the remeasurement process is performed under conditions which are identical or similar to the ID of the specimen and measurement conditions that are set before the remeasurement.

In step S11, the user is visually and/or audibly notified of the occurrence of a blood short sample (the notifying step: S11). At this time, as illustrated in Fig. 4B, the numerals of the mean corpuscular hemoglobin concentration can be clearly indicated by highlight on the displaying unit 90. The user may be notified by coloring (for example, in red) the numerals of the mean corpuscular hemoglobin concentration which are displayed on the displaying unit 90, and/or the background of the displaying unit 90. Alternatively, the user may be notified of the occurrence of a blood short sample by: coloring the display region for the specimen ID as illustrated in Fig. 9; displaying a message indicating an occurrence of a blood short sample; or sounding a buzzer.

Hereinafter, the reason why, when the mean corpuscular hemoglobin concentration is lower than the predetermined threshold, it is determined that a blood short sample occurs will be described.

A blood short sample often occurs particularly in the end of the suction step in which the blood in the blood collecting tube 10 is sucked by the suction tube 20. Namely, a blood short sample often occurs in the tip end side (on the side of the first region 241) of the blood analysis region 24. Here, the blood existing in the first region 241 of the blood analysis region 24 is subjected to the measurement of hemoglobin, and therefore the value of hemoglobin is caused to be low by a blood short sample. In the blood existing in the second region 242 of the blood analysis region 24, by contrast, a blood short sample does not occur, and therefore the hematocrit value which is measured by the second measuring unit 52 exhibits a normal value. In the case where the mean corpuscular hemoglobin concentration is reduced to be lower than the predetermined threshold, as a result, it is determined that a blood short sample occurs.

In the case where, even when the above-described remeasurement is repeatedly performed, the mean corpuscular hemoglobin concentration is lower than the predetermined threshold, it can be determined that the mean corpuscular hemoglobin concentration is not caused to be low by a blood short sample, and is originated from the specimen.

As described above, the blood analyzing method of the embodiment is a blood analyzing method in which, in the case where blood is sucked by the suction tube 20, a blood short sample in the suction tube 20 is detected. The blood analyzing method includes the determining step S10 in which, in the case where the mean corpuscular hemoglobin concentration of the blood in the blood analysis region 24 that is in the suction tube 20, and that is to be subjected to the blood analysis is lower than the predetermined threshold, it is determined that a blood short sample occurs. According to the blood analyzing method, a blood short sample in the suction tube 20 is detected by using the blood existing in the blood analysis region 24. Even in the case where a blood short sample occurs in the blood analysis region 24, therefore, the blood short sample can be detected. Consequently, a sample in which a blood short sample occurs in the blood analysis region 24 can be detected, and an erroneous measurement result can be prevented from being acquired.

As a method for detecting a blood short sample, for example, there is a method in which a ratio of hemoglobin and opacity or absorbance that are measured in different reservoirs is calculated, and, if the ratio is equal to or smaller than a predetermined threshold, it is determined that a short sample occurs. In this method, relatively large amounts of blood and reagents are required, and the measurement time period is prolonged. In the blood analyzing method of the embodiment, by contrast, a blood short sample is determined by using the mean corpuscular hemoglobin concentration. Therefore, an inspection can be performed by using relatively small amounts of blood and reagents, and the inspection time period can be shortened.

The blood analyzing method includes, before the determining step S10: the first measuring step S07 of measuring hemoglobin by using the blood existing in the first region 241 of the blood analysis region 24; the second measuring step S08 of measuring the red blood cell count and the hematocrit value by using the blood existing in the second region 242 that is in the blood analysis region 24, and that is different from the first region 241; and the calculating step S09 of calculating the mean corpuscular hemoglobin concentration based on the hemoglobin measured in the first measuring step S07, and the hematocrit value measured in the second measuring step S08. According to the blood analyzing method, hemoglobin and the hematocrit value are measured in the different regions 241, 242, and therefore it is possible to know in which region a blood short sample occurs.

The blood analyzing method may further include the notifying step S11 of, if it is determined in the determining step S10 that a blood short sample occurs, visually and/or audibly notifying of the blood short sample. According to the blood analyzing method, the user can be surely notified of the occurrence of a short sample.

In the notifying step S11, the blood short sample is visually notified by coloring the numerals of the mean corpuscular hemoglobin concentration which are displayed on the displaying unit 90. According to the blood analyzing method, the user can be more surely notified of the occurrence of a short sample.

In the notifying step S11, the blood short sample is visually notified by coloring the display region for the specimen and/or identification information of the subject which is displayed on the displaying unit 90. According to the blood analyzing method, the user can be more surely notified of the occurrence of a short sample.

If it is determined in the determining step S10 that a blood short sample occurs, blood is again sucked by the suction tube 20, and the determining step S10 is performed. According to the blood analyzing method, it is possible to acquire information of blood in a state where the blood is normally sucked.

In the case where blood is again sucked by the suction tube 20, and then the determining step S10 is performed, the determining step S10 is performed under measurement conditions which are identical or similar to those in the case where it is determined that a blood short sample occurs, or those which are arbitrarily set by the user. According to the blood analyzing method, it is possible to acquire information of blood in an easier manner.

As described above, the blood analyzing apparatus I of the embodiment detects a blood short sample in the suction tube 20 in the case where blood is sucked by the suction tube 20. The blood analyzing apparatus 1 includes the determining unit which, in the case where the mean corpuscular hemoglobin concentration of the blood in the blood analysis region 24 that is in the suction tube 20, and that is to be subjected to analysis of the blood is lower than the predetermined threshold, determines that a blood short sample occurs. According to the blood analyzing apparatus 1, a blood short sample in the suction tube 20 is detected by using the blood existing in the blood analysis region 24. Even in the case where a blood short sample occurs in the blood analysis region 24, therefore, the blood short sample can be detected. Consequently, a sample in which a blood short sample occurs in the blood analysis region 24 can be detected, and an erroneous measurement result can be prevented from being acquired.

The blood analyzing apparatus 1 further includes: the first measuring unit 51 which measures hemoglobin by using blood existing in the first region 241 of the blood analysis region 24; the second measuring unit 52 which measures the red blood cell count and the hematocrit value by using blood existing in the second region 242 that is in the blood analysis region 24, and that is different from the first region 241; and the calculating unit which calculates the mean corpuscular hemoglobin concentration based on the hemoglobin measured by the first measuring unit 51, and the hematocrit value measured by the second measuring unit 52. According to the blood analyzing apparatus 1, hemoglobin and the hematocrit value are measured in the different regions 241, 242, and therefore it is possible to know in which region a blood short sample occurs.

The present invention is not limited to the above-described embodiment, but can be variously modified within the scope of the appended claims.

For example, although, in the above-described embodiment, the white blood cell count of the sample in the third reservoir 43 is measured, hemoglobin of the sample in the first reservoir 41 is measured, and the red blood cell count and hematocrit value of the sample in the second reservoir 42 are measured, for example, the measurement order is not particularly limited.

According to the invention, the mean corpuscular hemoglobin concentration is calculated by using the hemoglobin measured in the first region 241 of the blood analysis region 24, and the hematocrit value measured in the second region 242. In an example not forming part of the invention, the mean corpuscular hemoglobin concentration may be calculated by using the hemoglobin and hematocrit value which are measured in the same region of the blood analysis region 24.

Although, in the above-described embodiment, if it is determined in the determining step S10 that a blood short sample occurs, blood is again sucked by the suction tube 20, and the determining step S10 is performed, the determining step S10 may not be again performed.

Although, according to the invention, the blood in the first region 241 is used in the measurement of hemoglobin, and that of the white blood cell count, and the blood in the second region 242 is used in the measurements of the red blood cell count and the hematocrit value, in an example not forming part of the invention the blood in the first region 241 may be used in the measurements of the red blood cell count and the hematocrit value, and the blood in the second region 242 may be used in the measurement of hemoglobin, and that of the white blood cell count. At this time, in the case where the HGB is reduced in the base end side (on the side of the second region 242) of the suction tube 20, and the mean corpuscular hemoglobin concentration is lower than the predetermined threshold, this can be detected as a blood short sample in the base end side of the suction tube 20.

According to an example not forming part of the invention, a configuration may be employed where the blood in the first region 241 is used as a blood parameter other than hemoglobin, the white blood cell count, the red blood cell count, and the hematocrit value (for example, the five-category classification of white blood cells), a third region may be disposed between the second region 242 and the second waste region 25, and blood in the third region may be used in the measurement of hemoglobin, and that of the white blood cell count. As described above, the first region and the third region can be interchanged with each other, and it may be possible to select which one of the basal side end and the tip end side is subjected to detection of a short sample.

## Claims

1. A blood analyzing method for detecting a short sample of blood in a suction tube (20) when blood is sucked by the suction tube (20), wherein a short sample of blood occurs when a required amount of blood is not sucked, the method comprising:
a first measuring step (S07) of measuring hemoglobin by using blood existing in a first region of a blood analysis region of the suction tube (20);
a second measuring step (S08) of measuring a red blood cell count and a hematocrit value by using blood existing in a second region that is in the blood analysis region, and that is different from the first region;
a calculating step (S09) of calculating the mean corpuscular hemoglobin concentration based on the hemoglobin measured in the first measuring step, and the hematocrit value measured in the second measuring step; and
a determining step (S10) of determining that a short sample of blood occurs, in a case where the mean corpuscular hemoglobin concentration is lower than a predetermined threshold.

2. The blood analyzing method according to claim 1 further comprising a notifying step (S11) of, if it is determined in the determining step that a short sample of blood occurs, visually and/or audibly notifying of an occurrence of the short sample of blood.

3. The blood analyzing method according to claim 2, wherein, in the notifying step (S11), the short sample of blood is visually notified by coloring numerals of the mean corpuscular hemoglobin concentration which are displayed on a displaying unit (90).

4. The blood analyzing method according to claim 2 or 3, wherein, in the notifying step (S11), the short sample of blood is visually notified by coloring a given display region for a specimen and/or identification information of a subject which is displayed on a displaying unit (90).

5. The blood analyzing method according to claim 1 further comprising a notifying step (S11) of, if it is determined in the determining step that a short sample of blood occurs, a command button of "REMEASUREMEMNT" is visually notified on a displaying unit (90).

6. The blood analyzing method according to claim 1, wherein, if it is determined in the determining step that a short sample of blood occurs, remeasurement of the blood is performed automatically.

7. The blood analyzing method according to any one of claims 1 to 4, wherein, if it is determined in the determining step that a short sample of blood occurs, blood is again sucked by the suction tube (20), and the determining step is performed.

8. The blood analyzing method according to claim 7, wherein, in a case where blood is again sucked by the suction tube (20), and then the determining step is performed, the determining step is performed under measurement conditions which are identical or similar to measurement conditions in a case where it is determined that a short sample of blood occurs, or measurement conditions which are arbitrarily set by a user.

9. A blood analyzing apparatus (1) for detecting a short sample of blood in a suction tube (20) when blood is sucked by the suction tube (20), wherein a short sample of blood occurs when a required amount of blood is not sucked, the apparatus comprising:
a first measuring unit (51) that measures hemoglobin by using blood existing in a first region of a blood analysis region of the suction tube (20);
a second measuring unit (52) that measures a red blood cell count and a hematocrit value by using blood existing in a second region that is in the blood analysis region, and that is different from the first region;
a calculating unit that calculates the mean corpuscular hemoglobin concentration based on the hemoglobin measured by the first measuring unit (51), and the hematocrit value measured by the second measuring unit (52); and
a determining unit that determines that a short sample of blood occurs in a case where the mean corpuscular hemoglobin concentration is lower than a predetermined threshold.

10. The blood analyzing apparatus (1) according to claim 9 further comprising a displaying unit (90), the apparatus configured to visually notify a command button of "REMEASUREMEMNT" on the displaying unit (90) if it is determined in the determining step that a short sample of blood occurs.

11. The blood analyzing apparatus (1) according to claim 9, configured to automatically perform remeasurement of the blood if it is determined that a short sample of blood occurs,.

## Patentansprüche

1. Blutanalyseverfahren zum Ermitteln einer kurzen Blutprobe in einem Saugrohr (20), wenn Blut durch das Saugrohr (20) gesaugt wird, wobei eine kurze Blutprobe auftritt, wenn eine erforderliche Blutmenge nicht angesaugt wird, wobei das Verfahren umfasst:
einen ersten Messschritt (S07) zum Messen von Hämoglobin unter Verwendung von Blut, das in einem ersten Bereich eines Blutanalysebereichs des Saugrohrs (20) vorhanden ist;
einen zweiten Messschritt (S08) zum Messen einer Gesamtzahl der roten Blutkörperchen und eines Hämatokritwerts unter Verwendung von Blut, das in einem zweiten Bereich vorhanden ist, der sich in dem Blutanalysebereich befindet und sich von dem ersten Bereich unterscheidet;
einen Berechnungsschritt (S09) zum Berechnen der mittleren korpuskularen Hämoglobinkonzentration auf der Grundlage des im ersten Messschritt gemessenen Hämoglobins und des im zweiten Messschritt gemessenen Hämatokritwerts; und
einen Bestimmungsschritt (S10) zum Bestimmen, dass eine kurze Blutprobe auftritt, wenn die mittlere korpuskulare Hämoglobinkonzentration unter einem vorgegebenen Schwellenwert liegt.

2. Blutanalyseverfahren nach Anspruch 1, des Weiteren umfassend einen Meldeschritt (S11), bei dem, wenn in dem Bestimmungsschritt bestimmt wird, dass eine kurze Blutprobe auftritt, das Auftreten der kurzen Blutprobe optisch und/oder akustisch gemeldet wird.

3. Blutanalyseverfahren nach Anspruch 2, wobei in dem Meldeschritt (S11) die kurze Blutprobe durch Einfärben von Ziffern der mittleren korpuskularen Hämoglobinkonzentration, die auf einer Anzeigeeinheit (90) angezeigt werden, visuell gemeldet wird.

4. Blutanalyseverfahren nach Anspruch 2 oder 3, wobei in dem Benachrichtigungsschritt (S11) die kurze Blutprobe durch Einfärben eines gegebenen Anzeigebereichs für eine Proben- und/oder Identifikationsinformation eines Subjekts, die auf einer Anzeigeeinheit (90) angezeigt wird, visuell gemeldet wird.

5. Blutanalyseverfahren nach Anspruch 1, des Weiteren umfassend einen Meldeschritt (S11), bei dem, wenn in dem Bestimmungsschritt bestimmt wird, dass eine kurze Blutprobe auftritt, eine Befehlstaste "REMEASUREMENT"(NEU MESSEN) auf einer Anzeigeeinheit (90) visuell angezeigt wird.

6. Blutanalyseverfahren nach Anspruch 1, wobei, wenn in dem Bestimmungsschritt bestimmt wird, dass eine kurze Blutprobe auftritt, eine erneute Messung des Blutes automatisch durchgeführt wird.

7. Blutanalyseverfahren nach einem der Ansprüche 1 bis 4, wobei, wenn in dem Bestimmungsschritt bestimmt wird, dass eine kurze Blutprobe auftritt, erneut Blut durch das Saugrohr (20) gesaugt wird und der Bestimmungsschritt durchgeführt wird.

8. Blutanalyseverfahren nach Anspruch 7, wobei in einem Fall, in dem erneut Blut durch das Saugrohr (20) gesaugt und dann der Bestimmungsschritt durchgeführt wird, der Bestimmungsschritt unter Messbedingungen durchgeführt wird, die identisch oder ähnlich den Messbedingungen in einem Fall sind, in dem bestimmt wird, dass eine kurze Blutprobe auftritt, oder unter Messbedingungen, die von einem Benutzer willkürlich eingestellt werden.

9. Blutanalysevorrichtung (1) zum Ermitteln einer kurzen Blutprobe in einem Saugrohr (20), wenn Blut durch das Saugrohr (20) gesaugt wird, wobei eine kurze Blutprobe auftritt, wenn eine erforderliche Blutmenge nicht angesaugt wird, wobei die Vorrichtung umfasst:
eine erste Messeinheit (51), die Hämoglobin unter Verwendung von Blut misst, das in einem ersten Bereich eines Blutanalysebereichs des Saugrohrs (20) vorhanden ist;
eine zweite Messeinheit (52), die eine Gesamtzahl der roten Blutkörperchen und einen Hämatokritwert unter Verwendung von Blut misst, das in einem zweiten Bereich vorhanden ist, der sich in dem Blutanalysebereich befindet und sich von dem ersten Bereich unterscheidet;
eine Berechnungseinheit, welche die mittlere korpuskulare Hämoglobinkonzentration auf der Grundlage des von der ersten Messeinheit (51) gemessenen Hämoglobins und des von der zweiten Messeinheit (52) gemessenen Hämatokritwerts berechnet; und
eine Bestimmungseinheit, die bestimmt, dass eine kurze Blutprobe auftritt, wenn die mittlere korpuskulare Hämoglobinkonzentration unter einem vorgegebenen Schwellenwert liegt.

10. Blutanalysevorrichtung (1) nach Anspruch 9, des Weiteren umfassend eine Anzeigeeinheit (90), wobei die Vorrichtung dazu konfiguriert ist, eine Befehlstaste "REMEASURE-MENT" (NEU MESSEN) auf der Anzeigeeinheit (90) visuell anzuzeigen, wenn in dem Bestimmungsschritt bestimmt wird, dass eine kurze Blutprobe auftritt.

11. Blutanalysevorrichtung (1) nach Anspruch 9, die dazu konfiguriert ist, automatisch eine erneute Messung des Blutes durchzuführen, wenn bestimmt wird, dass eine kurze Blutprobe vorliegt.

## Revendications

1. Procédé d'analyse de sang pour détecter un échantillon réduit de sang dans un tube d'aspiration (20) lorsque le sang est aspiré par le tube d'aspiration (20), dans lequel un échantillon réduit de sang se présente lorsqu'une quantité requise de sang n'est pas aspirée, le procédé comprenant :
une première étape de mesure (S07) de mesure d'une hémoglobine en utilisant du sang présent dans une première zone d'une zone d'analyse de sang du tube d'aspiration (20) ;
une deuxième étape de mesure (S08) de mesure d'un compte de globules rouges et d'une valeur d'hématocrite utilisant le sang présent dans une deuxième zone qui se trouve dans la zone d'analyse de sang, et qui est différente de la première zone ;
une étape de calcul (S09) de calcul de la concentration corpusculaire moyenne en hémoglobine selon l'hémoglobine mesurée à la première étape de mesure, et la valeur d'hématocrite mesurée à la deuxième étape e mesure ; et
une étape de détermination (S10) de détermination qu'un échantillon réduit de sang se présente, dans un cas où la concentration corpusculaire moyenne en hémoglobine est inférieure à un seuil prédéterminé.

2. Le procédé d'analyse de sang selon la revendication 1 comprenant en outre une étape de notification (S11), s'il est déterminé à l'étape de détermination qu'un échantillon réduit de sang se présente, de notification visuelle et/ou auditive d'une présence de l'échantillon réduit de sang.

3. Le procédé d'analyse de sang selon la revendication 2, dans lequel, à l'étape de notification (S11), l'échantillon réduit de sang est visuellement notifié par une coloration de chiffres de la concentration corpusculaire moyenne en hémoglobine qui sont affichés sur une unité d'affichage (90).

4. Le procédé d'analyse de sang selon la revendication 2 ou 3, dans lequel, à l'étape de notification (S11), l'échantillon réduit de sang est visuellement notifié par une coloration d'une zone d'affichage donnée pour un spécimen et/ou des informations d'identification d'un sujet qui s'affichent sur une unité d'affichage (90).

5. Le procédé d'analyse de sang selon la revendication 1 comprenant en outre une étape de notification (S11), s'il est déterminé à l'étape de détermination qu'un échantillon réduit de sang se présente, d'une notification visuelle d'un bouton de commande de remesure (« REMEASUREMENT ») sur une unité d'affichage (90) .

6. Le procédé d'analyse de sang selon la revendication 1, dans lequel, s'il est déterminé à l'étape de détermination qu'un échantillon réduit de sang se présente, une remesure du sang s'exécute automatiquement.

7. Le procédé d'analyse de sang selon l'une quelconque des revendications 1 à 4, dans lequel, s'il est déterminé à l'étape de détermination qu'un échantillon réduit de sang se présente, le sang est à nouveau aspiré par le tube d'aspiration (20), et l'étape de détermination est exécutée.

8. Le procédé d'analyse de sang selon la revendication 7, dans lequel, dans un cas où le sang est à nouveau aspiré par le tube d'aspiration (20), et puis l'étape de détermination est exécutée, l'étape de détermination est exécutée selon des conditions de mesure qui sont identiques ou similaires à des conditions de mesure d'un cas où il est déterminé qu'un échantillon réduit de sang se présente, ou des conditions de mesure qui sont arbitrairement définies par un utilisateur.

9. Dispositif d'analyse de sang (1) pour détecter un échantillon réduit de sang dans un tube d'aspiration (20) lorsque le sang est aspiré par le tube d'aspiration (20), dans lequel un échantillon réduit de sang se présente lorsqu'une quantité requise de sang n'est pas aspirée, le dispositif comprenant :
une première unité de mesure (51) qui mesure une hémoglobine en utilisant du sang présent dans une première zone d'une zone d'analyse de sang du tube d'aspiration (20) ;
une deuxième unité de mesure (52) qui mesure un compte de globules rouges et une valeur d'hématocrite en utilisant le sang présent dans une deuxième zone qui se trouve dans la zone d'analyse de sang, et qui est différente de la première zone ;
une unité de calcul qui calcule la concentration corpusculaire moyenne en hémoglobine selon l'hémoglobine mesurée par la première unité de mesure (51), et la valeur d'hématocrite mesurée par la deuxième unité de mesure (52) ; et
une unité de détermination qui détermine qu'un échantillon réduit de sang se présente dans un cas où la concentration corpusculaire moyenne en hémoglobine est inférieure à un seuil prédéterminé.

10. Le dispositif d'analyse de sang (1) selon la revendication 9 comprenant en outre une unité d'affichage (90), le dispositif configuré pour notifier visuellement un bouton de commande de remesure (« REMEASUREMENT ») sur l'unité d'affichage (90) s'il est déterminé à l'étape de détermination qu'un échantillon réduit de sang se présente.

11. Le dispositif d'analyse de sang (1) selon la revendication 9, configuré pour exécuter automatiquement une remesure du sang s'il est déterminé qu'un échantillon réduit se présente.
